(19) Europäisches Patentamt / European Patent Office / Office européen des brevets

(11) **EP 1 173 093 B1**

(12) **EUROPEAN PATENT SPECIFICATION**

(45) Date of publication and mention
of the grant of the patent:
**25.09.2013 Bulletin 2013/39**

(21) Application number: **00929424.0**

(22) Date of filing: **26.04.2000**

(51) Int Cl.:
***A61B 5/029*** (2006.01)

(86) International application number:
**PCT/EP2000/003697**

(87) International publication number:
**WO 2000/064339 (02.11.2000 Gazette 2000/44)**

(54) **METHOD AND APPARATUS FOR MEASURING CARDIAC FLOW OUTPUT**

VERFAHREN UND GERÄT ZUR BESTIMMUNG DES HERZZEITVOLUMENS

PROCÉDÉ ET APPAREIL DE MESURE DU DÉBIT CARDIAQUE

(84) Designated Contracting States:
**AT BE CH CY DE DK ES FI FR GB GR IE IT LI LU
MC NL PT SE**

(30) Priority: **27.04.1999 IT FI990098**

(43) Date of publication of application:
**23.01.2002 Bulletin 2002/04**

(60) Divisional application:
**10011456.0 / 2 329 765**

(73) Proprietor: **Romano, Salvatore**
**50144 Firenze (IT)**

(72) Inventor: **Romano, Salvatore**
**50144 Firenze (IT)**

(74) Representative: **Scilletta, Andrea**
**Via Antonio Salandra, 18**
**00187 Roma (IT)**

(56) References cited:
**EP-A- 0 947 160      WO-A-99/02086**
**US-A- 5 400 793      US-A- 5 647 369**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

**Description**

**Field of the invention**

The present invention refers to a method and an apparatus for determining the stroke volume - i.e., the volume of blood expelled from the left ventricle (LSV), the volume of blood expelled from the right ventricle (RSV) - and hence the cardiac output Q - i.e., the stroke volume multiplied by the heart rate (HR) -, in a continuous way, using invasive and non-invasive indirect techniques, so as to enable acquisition of this important haemodynamic parameter in various clinical and non-clinical situations, as well as in the course of ergometric tests.

**Prior art**

For the measurement of cardiac output Q, the invasive methods that are currently most extensively used are the Thermodilution Method (TDM), Fick's Method (FM), and a method that uses the arterial pressure signal p(t) measured in the aorta or in the pulmonary artery, referred to as the Pulse Contour Method (PCM).
This method which uses the signal pressure is not very reliable and for this reason it is necessary to make a calibration. This is usually the TDM. At the present time reliable results have not been attained by this method.
This PCM method derives from an original idea of Herd [Herd J.A. *et al*., 1864] and from the theory referred to as the Windkassel (German for "air chamber") theory of Franck (Franck O., 1930), and is based on the existence of a relationship between the volume of blood expelled by the left ventricle (LSV) or the volume of blood expelled by the right ventricle (RSV ), and the area under the pressure curve p (t) . The fundamental relation used for calculating the stroke volume is SV=A/Z0 where A, expressed in [mmHg t], is the area under the pressure curve p (t) (see figure A1), and Z0, expressed in [mmHg/cm/t], is the hydraulic impedance which depends upon the dynamic resistances and upon the compliance of the artery wall. LSV is measured in [cm³], hence Q = LSV*HR is the cardiac output expressed in litres per minute if the heart rate is measured in beats per minute. In this connection we recall that the plot of the arterial pressure with respect to time is determined by the magnitude of LSV and by the vascular impedance. Consequently, the Pulse Contour Method endeavours to separate and analyse these two contributions; however, the method is unable to determine the two contributions as independent variables over time.
Applying the Windkassel theory, many studies have attempted to determine LSV only from the pressure waveform and from the characteristics linked to transmission of the wave in the aorta or in the pulmonary artery (Remington J.W. *et al.,* 1948; Warner H.R. *et al.*, 1953; Herd J.A. *et al.*, 1966; Kouchoukos N.T. *et al.*, 1970].
The original idea of Franck has subsequently been applied over the years and has made it possible to estimate LSV in a continuous way from the measurement of the pressure signal in the aorta or in the pulmonary artery [McDonald D.A. *et al.*, 1974; Wesseling K.H. *et al*., 1976; Tajimi T. *et al*., 1983; Wesseling K.H. *et al.*, 1993].
However, in the concrete application to the various possible clinical situations, the Pulse Contour Method currently requires a "calibration" for calculation of the hydraulic impedance. For the calibration, generally one of the other two methods referred to above, i.e., the thermodilution method and Fick's method, is used, or else linear regressions of aortic parameters, such as the diameter of the aorta, and the age, sex, height and weight of the patient are used.
Unfortunately, the calibration and regression factors are subject to error, given that the methods from which they are obtained are in turn imprecise and that the regressions are in any case obtained on a limited number of subjects, and are hence acceptable only as a mean and not as a true measurement of the quantity investigated.
In fact, cardiac output estimated using the thermodilution method and Fick's method are not always in agreement with the clinical parameters obtained using other diagnostic techniques, and this mainly occurs in patients suffering from certain forms of heart disease, such as disease involving dilatation of the heart, valvular cardiopathy, and cardiac fibrillation.
To provide an example, consider two possible signals in the aorta studied between the points of opening and closing of the ventricle. These signals will in general present the same area but different forms, with different times of attainment of the systolic point.
The traditional Pulse Contour Method will therefore yield the same exact measurement (same integral) evaluated on the basis of the calibration impedance. However, it is evident that from a different form of the signal there must derive a different impedance, which cannot be evaluated. US Patent No. US 5 647 369 discloses a method that calculates a measure of cardiac output based on the patient's blood pressure.
Hence, the limits of the invasive techniques currently in use are: a) the poor precision achievable in the estimation of cardiac output on account of clinical illnesses; b) the non- applicability in general on account of the pathological condition of the patient; and c) the impossibility of applying the said invasive techniques, for example during an ergometric test.

**Scope of the invention**

A first scope of the invention is to obtain measurements in a continuous way that are more reliable than the ones obtained using the invasive and non-invasive techniques currently applied.

A second scope is to render the measurement substantially independent of the point of application of the sensor by introducing variations in the specific formulas, without any need for prior calibration of the measurement.

**Summary of the invention**

The above purposes have been achieved according to the invention using a method which directly obtain the cardiac output from the pressure signal measured in an invasive way, in the ascending aorta, in the pulmonary artery and in femoral brachial and radial, or measured in a non-invasive way, for example from the arteriole of the finger using a cuff meter. According to the method, the impedance of the pressure signal is calculated on the basis of the points of resonance of the signal by assimilating the signal to that of a flow in an elastic tube and assuming constancy of Young's modulus, which is in particular taken to have a value of unity. In this way, it is possible to calculate the cardiac output without any longer having to use various calibrations, but exclusively from an analysis of the pressure wave and its characteristics.

The hydraulic impedance is calculated by means of an analysis of the first and second derivatives with respect to time of the pressure signal recorded.

According to a further aspect of the invention, a correction is moreover made of the mean pressure value to be used for the calculation of LSV in order to take into account the attenuations of the said value in the various points of possible recording of the signal.

According to a further aspect of the invention, from the signal recorded in the finger (or from some other point in a non-invasive way), the method makes possible a direct reconstruction of the signal in the aorta and in the pulmonary artery, and from the latter signal a reconstruction of the cardiac output.

More specifically, according to the invention in order to obtain the SV estimation we have taken into consideration the wave pressure in the ascending aorta and/or in pulmonary artery, the artery compliance (E) and the periferical resistance (R). Therefore we have taken into consideration that 1) the SV depends on the pressure variation obtained at the opening of the ventricular valve (that is the difference between systolic and diastolic pressure divided by the time necessary to pass between the systolic and diastolic) and 2) the SV is conditioned by E and R.

In order to obtain these contributions we have taken into account the value of the dicrotic pressure and the other characteristic points between the systolic dicrotic pressure (this pressure values must be divided by time. This time is the difference between the end of the cardiac beat and the time of the event being taken into consideration).

Therefore we have considered SV as being determined by three points: 1) the bolus of blood ejected by the ventricular; 2) the reaction due to the aortic wall; 3) resistance due to periferic arterial cycle. As the value of the pressure at the point where it is taken is the result of these three components at the same time we have studied our system in a perturbative way. Therefore we have considered the principle contribution of the ventricular and of the system E and R, the first being given by 1) as described above and the second, the E and R system principally contributes to the closure of the valve ( point of diacrotic pressure) This last event point is conditioned by a series of perturbations on the pressure signal after the cardiac valve, according to the vase being crossed and the length of the course. That is it is necessary to take into consideration not only the contribution due to the systolic and to the diacrotic above described, and when present those due to secondary perturbations.

In conclusion all the event points which have been taken into consideration are the moments in which there is a state of balance between the various points (blood ejected from the ventricular- E- R) : the "principle" balancing points ( systolic and dicrotic points) can be or not "accompanied" by other balancing points (how and if to analyse them will be described later) . All this information can be found in the wave pressure which flows after having been generated by the ventricular (right or left) .

Advantageously, with the method according to the invention it is possible to establish a relationship between the hydraulic impedance and the usable time, also in combination with known methods (e.g., the thermodilution method) which involve a phase of calibration of the signal recorded, where the contribution of the area under the pressure curve is considered variable over time, whilst the contribution of the impedance can only be considered as constant.

In particular, by the method of the present invention (hereinafter called pulse analytical method (PAM)) it has been possible: a) to find the SV from the signal pressure recorded invasively in ascending aorta and in pulmonary artery; b) to find the SV from the arterial signal pressure invasively recorded (brachial, radial, and femoral artery) and non invasively recorded (e.g. from the pressure obtained with the oscillometric method from the arterial finger).

In this way we estimated the LSV and so we determined the true Q in a way that is completely free from any calibration. Therefore these results are obtained only by the analysis of the wave pressure ( depending only on where the wave pressure was taken).

According to the invention an apparatus able to perform the method is provided. The apparatus comprises a microprocessor unit able to receive the blood pressure signal and to analyse it over the time in order to determine the above identified parameters and to calculate the cardiac output Q therefrom.

In a preferred embodiment the apparatus further comprises a sensor in the shape of a cuff meter able to be applied to a finger and to acquire the blood pressure signal. The invention is defined in claims 1, 4, and 6. Any embodiment, which is in contradiction to these claim's is not part of the invention.

**Brief description of the figures**

-    Figure A1 shows a cardiac pressure signal diagram as analysed by the prior art method;
-    Figures 1-19 show the form of the cardiac pressure signal and of its first and  second derivatives, the signal being taken in various recording points;

-    Figure 20 shows a reconstruction, according to the present method, of the signal in the aorta starting from the pressure signal recorded at the arteriole of the finger.

**Detailed description of the invention**

In the following, pressure is expressed in the unit "mmHg", which has the following conversion relationship with the appropriate Si unit "Pascal": 1mmHg = (101325/760) Pascal.

With reference to the annexed figures, various examples of application of the method are described in what follows.

Example I

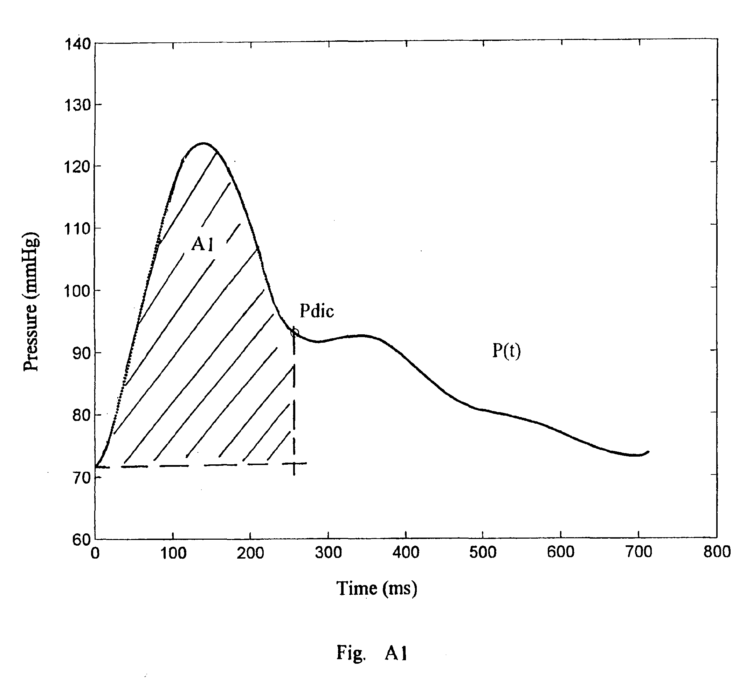

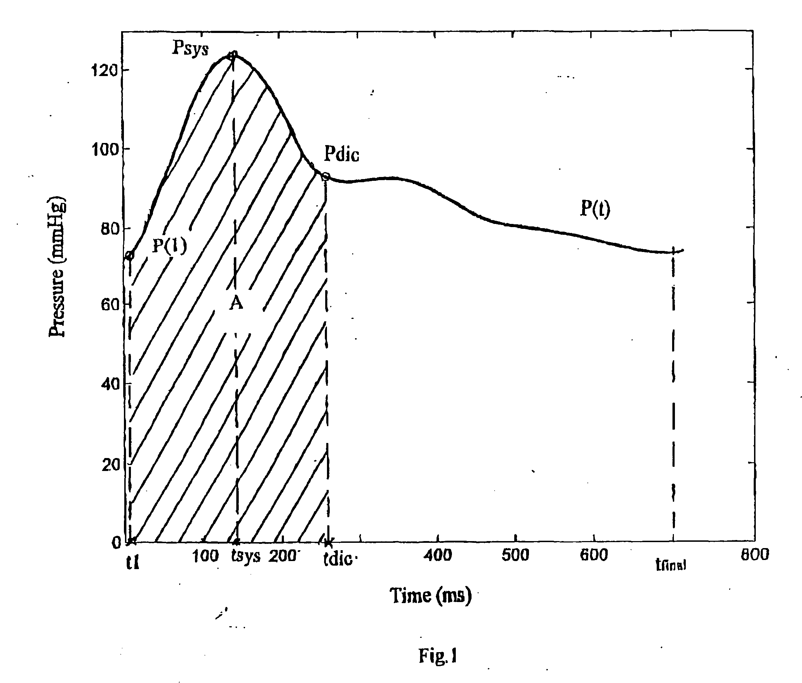

A) Relation between LSV and pressure taken in the ascending aorta (Pulse Analytical Method, Aortic; PAMA) (Fig.1-6)

i) The PAMA determines the heart flow Q in litres per minute using the following general relation (the pressure signal is acquired in the ascending aorta at 1000 Hz):

$$LSV = [K[A/((Za1+Za2)*1000)+A/((Za1+Za2)*1000)*(Pm-K1)/K1]]/1000$$

$$Eq. [1]$$

where:

K = 1 and has the dimensions [($\lambda$m*sqrt (2p/ (p) *Vm], expressed in [$l^3/t^2$] ;
$\lambda$m is the mean wavelength, approximately equal to 10 m
Vm is the mean velocity, approximately equal to 10 m/s
p is the blood density;
A is the integral between t1 (time at the diastolic pulse in [ms]) and tdic (time at the dicrotic pressure in [ms]) under the pressure curve p(t), expressed in [mmHg*ms] (Figure 1);
K1 = 100, expressed in [mmHg], and represents the correction factor of the mean pressure;
Za1 = (psys- p (1) ) / tsys, expressed in [mmHg/ms] ;
Za2 = (pdic/tfinal - tdic), expressed in [mmHg/ms]; and
Pm = (psys+2p (1) ) / 3. see the following Note1.
tfinal= time of the beat being taken into consideration (time origin in t1 and final in final)

The cardiac output is thus

$$Q = LSV*HR$$

where Q is expressed in [lit/min];
HR = 60000/T; and
T is the cardiac period expressed in [ms].

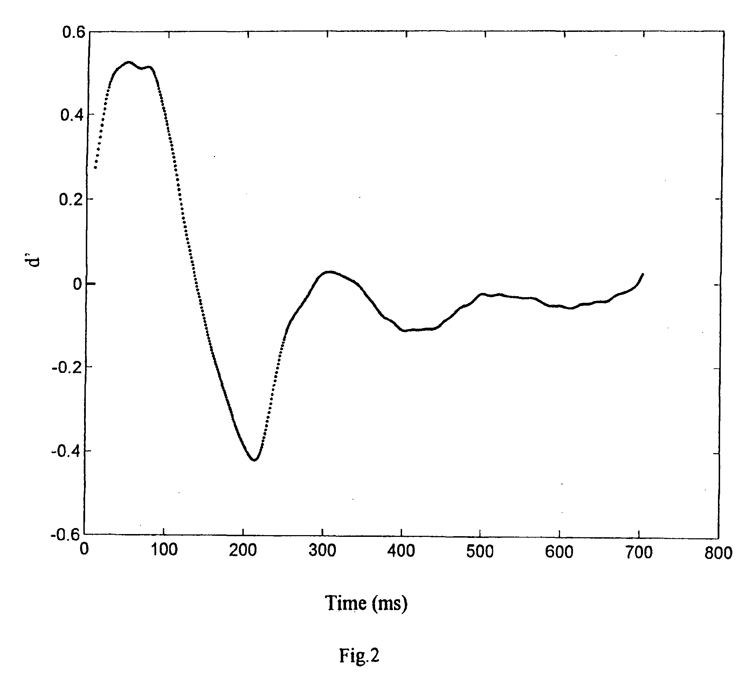

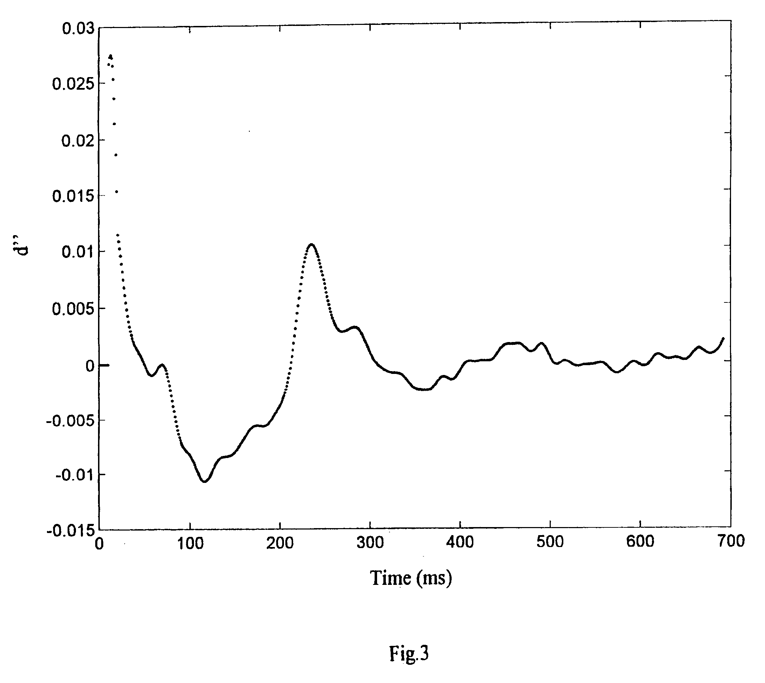

This relation was applied in cases In which the pressure curve and the corresponding mean of the tangent at 21 points (i.e., the first derivative d') and the mean of the tangent at 21 points of the mean tangent (i.e., the second derivative d") were those shown in Figures 2 and 3 and could be associated to the recording point.

ii) With -Za3

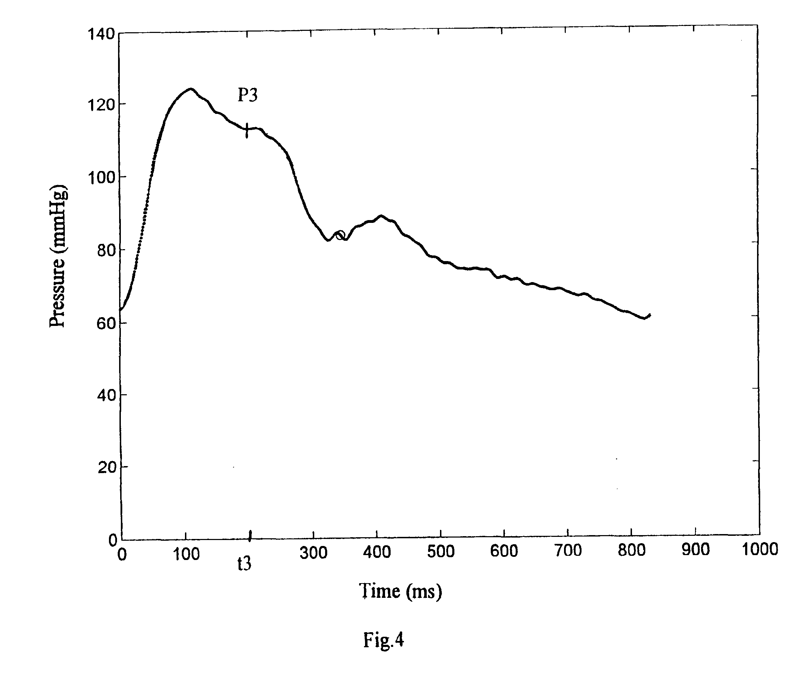

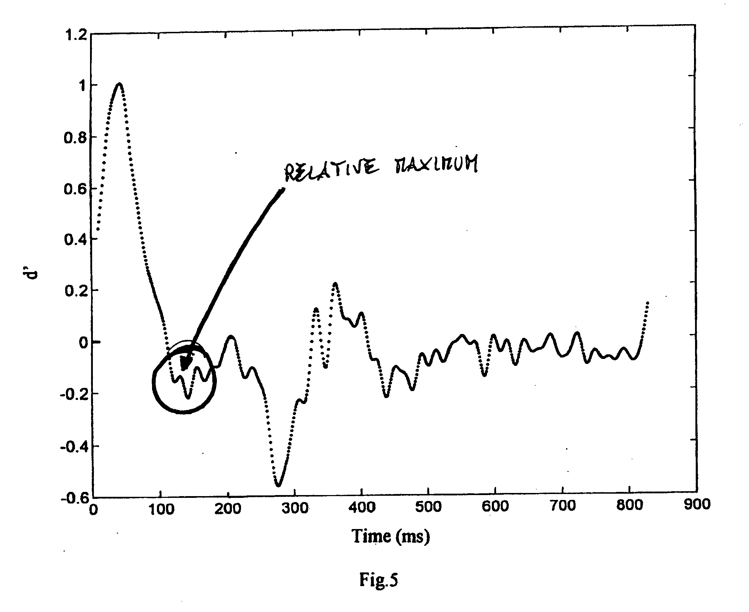

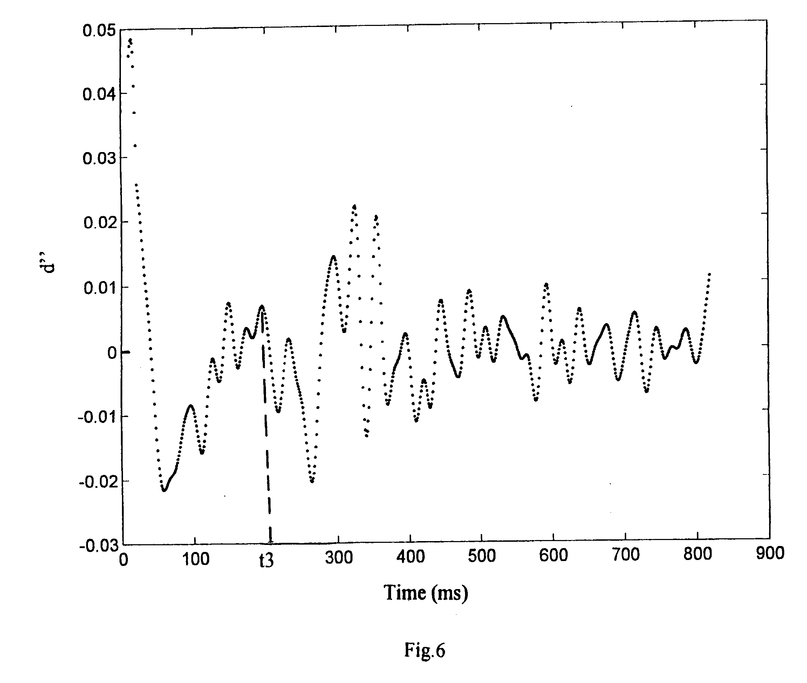

In the cases where the pressure curves in the ascending aorta were of the type shown In Figure 4, and the corresponding first and second derivatives, d' and d", were as those shown in Figures 5 and 6 and revealed the point of resonance at time t3, then the relation became:

$$LSV = [K[A/((Za1+Za2-Za3)*1000)+A/((Za1+Za2-Za3)*1000)*(Pm-K1)/K1]] /1000$$

$$Eq.[2]$$

where the symbols have the same meaning as in Eq. [1], and where t3 is the time, in [ms], at the minimum value of d" between the time tsys and the time tdic, and p3 is the corresponding pressure expressed in [mmHg] at time t3 (see Figure 6) and Za3 = (P3/tfinal - t3) mmHg/ms

In a similar way it is possible to calculate Q = LSV*HR.

Note 1

The mean pressure for the pressure measured in the ascending aorta must be considered as such for the interval 90 - 110 mmHg; for the mean pressure between 110 - 120 and 90 - 80 mmHg it must be considered at 50% (for example for a Pm= 118 mmHg for our method is = 114 mmHg); for mean pressure values between 120 - 130 and 80 - 70 mmHg it must be considered at 25%, for mean pressure values>=130 and <=70 mmHg it must be considered 13%

Example II

B) Relation between RSV and the pressure taken in the pulmonary artery (Pulse Analytical Method, Pulmonary: PAMP)

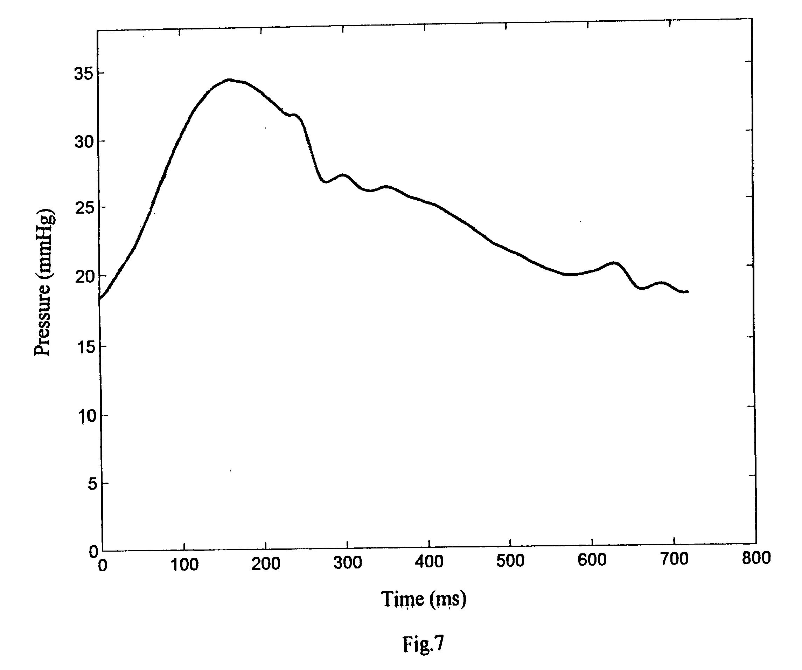

Relationship between the volume of blood expelled from the right ventricle RSV and the pressure measured in the pulmonary artery. The corresponding signal pressure is similar to the one represented for aortic pressure, but for variations in scale (see Figure 7).

The PAMP determines the heart flow Q in litres per minute using the following general relation (the pressure is acquired in the pulmonary artery at 1000 Hz):

i) Case with mean pressure in pulmonary artery >= 19mmHg

$$RSV = [K[A/((Za1+Za2)*1000)+A/((Za1+Za2)*1000)*(Pm-K1)/K1]]/1000 \quad [Eq.3]$$

where:

K = 1 and has the dimensions [($\lambda_m$*sqrt (2p/ ($\rho$) *Vm), expressed in [$I^3/ t^2$], $\rho$ being the density of the blood;
A is the integral between t1 (time at the diastolic pulse in [ms]) and tdic (time at the second dilatation of the artery in a dicrotic pulse in [ms]) under the pressure curve p(t), expressed in [mmHg*ms];
K1 = 12, expressed in [mmHg];
Za1 = (psys) / tsys, expressed in [mmHg/ms] ;
Za2 = (pdic/ tfinal- tdic), expressed in [mmHg/ms] ; and
Pm = (psys+2p (1) / 3) ; see following Note 2

$$Q = RSV*HR,$$

where Q is expressed in [lit/min];

HR = 60000/T; and
T is the cardiac period expressed in [ms].

In Figure 7 a signal acquisition of the pressure in the pulmonary artery is shown. For the pressure in pulmonary artery we have variations for d' and d" similar to those of the aorta. In consequence the determination of the point of dicrotic pressure (Pdic), the systolic pressure (Psys), diastolic pressure (P (1) ) and the relative times is as described above.

ii) Case with mean pressure in pulmonary artery <= 19mmHg In the cases in which Pm is <= 19 mmHg the relation becomes:

$$RSV=[K [ A / ((Za1+Za2)*1000)+A / ((Za1+Za2)*1000]] / 1000 \qquad \text{Eq.[4]}$$

With the same meaning for the symbols as in the preceding cases. In the same way it is possible to calculate Q=RSV*HR

Note 2

The mean pressure in the case of pressure taken in the pulmonart artery must be taken as such for the interval of pressure between 19 - 28 mmHg; for values of  mean pressure between 28 - 33 mmHg it must be considered at 50%, for values of mean pressure > 33mmHg it must be considered at 25% (for example a pm=43mmHg for our method Is equal to 33 mmHg); for values < 19mmHg we are in case ii) and therefore we do not use the mean pressure.

Example III

C) Relation between LSV and the pressure non invasively recorded from the arterial finger (Pulse Analysis Method, Finger: PAMF)

Direct relationship

i) The PAMF determines the cardiac output Q in litres per minute using the following general relation (the pressure is acquired at the finger of the left hand at 1000 Hz):

$$LSV = [k[A/((Zf1+Zf2)*1000)+A/((Zf1+Zf2)*1000)*(Pm-K1)/K1]]/1000 \qquad \text{Eq. [5]}$$

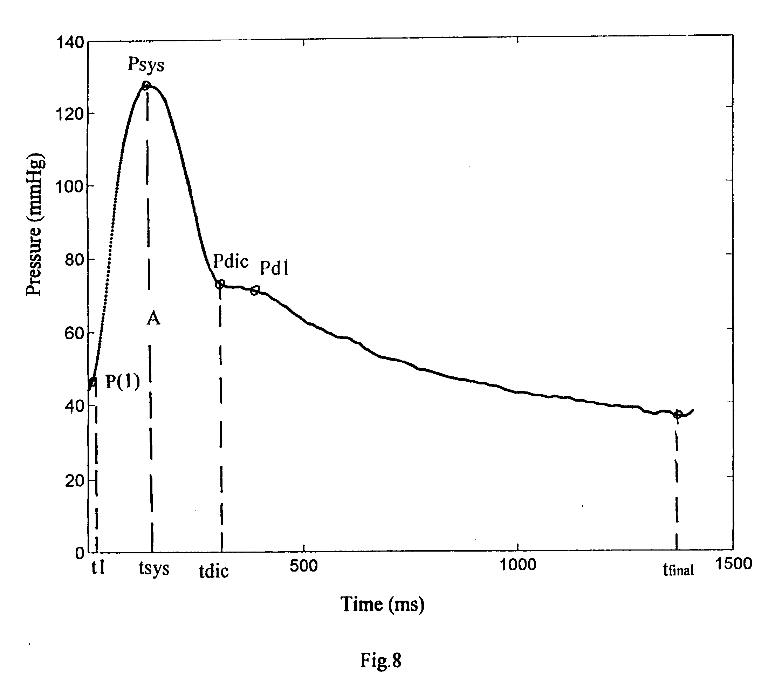

where (Figure 8):

K = 1 and has the dimensions [($\lambda$m*sqrt (2p/ ($\rho$) *Vm] expressed in [$l^3/ t^2$] ;
A is the integral between t1 (time at the diastolic pulse in [ms]) and tdic (time at the dicrotic pressure in [ms]) under the pressure curve p(t), expressed in [mmHg*ms];
K1 = 90, expressed in [mmHg];
Zf1 = (psys- p (1) ) / tsys, expressed in [mmHg/ms] ;
Zf2 = pdic/(tfinal - tdic), expressed in [mmHg/ms]; and
Pm = (psys+2p (1) ) / 3.

see following Note 3
The corrected volume of blood expelled from the left ventricle (LSVC) is

$$LSVC = [LSV+LSV*abs(delta(Pd1-pdic))/(psys-pdias)] \qquad [6]$$

where:

(Pd1- pdic) = variation of pressure of the dicrotic point (Pdic) at its maximum (Pd1) = [mmHg] . This correction exists only when there is an increase in the pressure after the dicrotic pressure ((Pd1- Pdic) >0) . In the cases in which the increase in pressure is not present ((Pd1- Pdic) <=0) we have LSV=LSVC.

Psys Is the systolic pressure, expressed in [mmHg];
Pdias is the diastolic pressure, expressed in [mmHg]; and

the term Pd1 is calculated immediately after the dicrotic point and is the maximum value of the curve after (Pdic).

$$Q = LSVC^*HR$$

where Q is expressed in [lit/min];
HR = 60000/T; and
T is the cardiac period expressed in [ms].

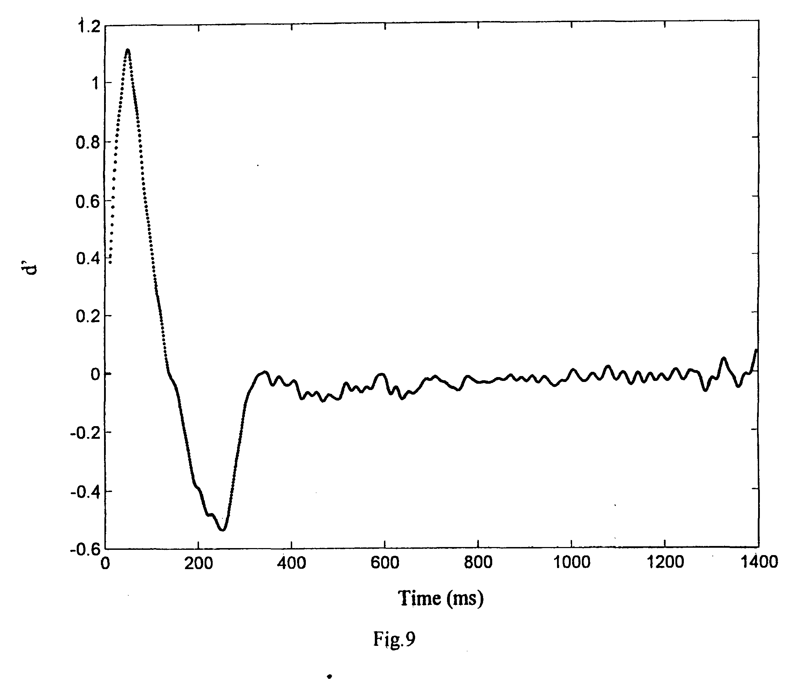

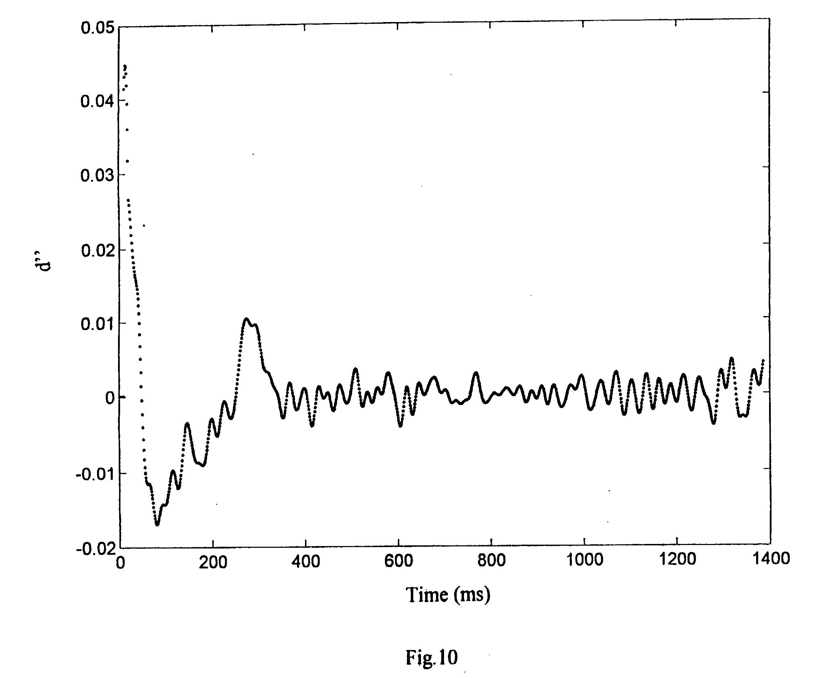

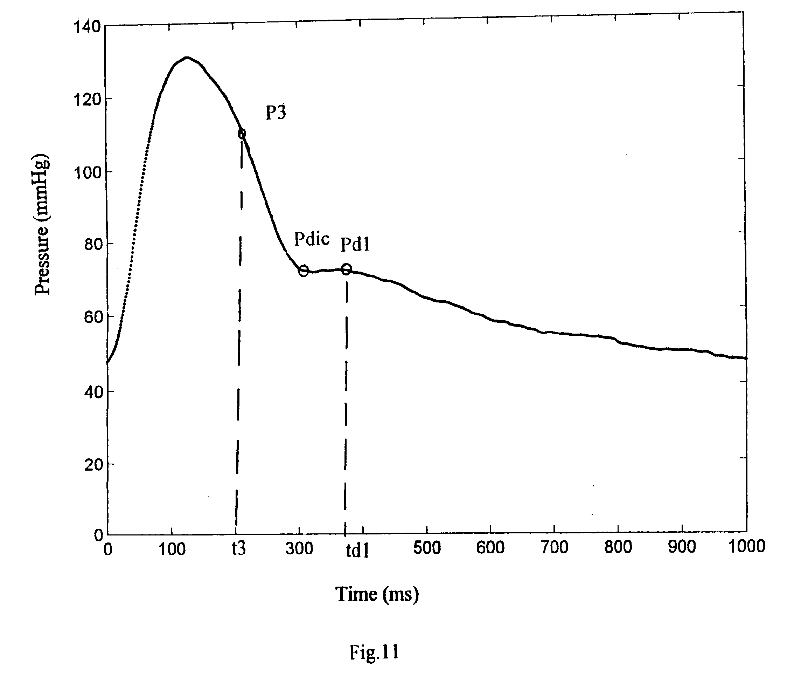

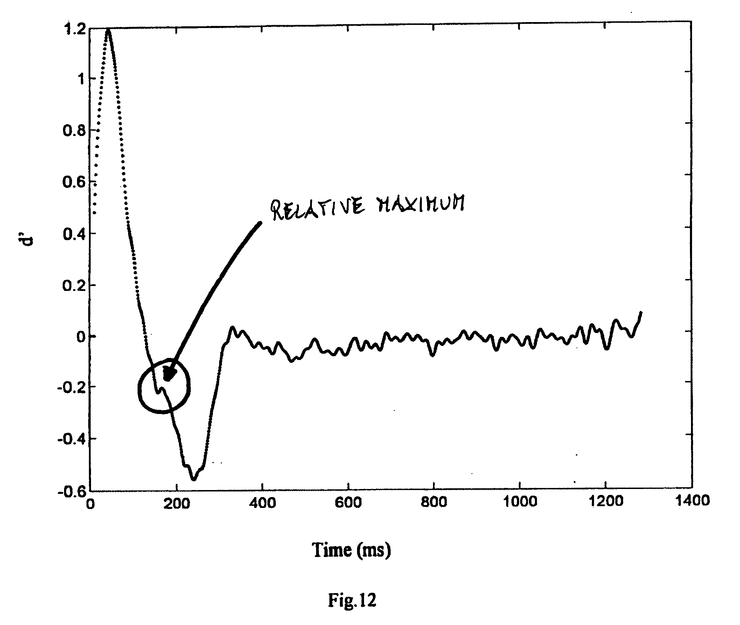

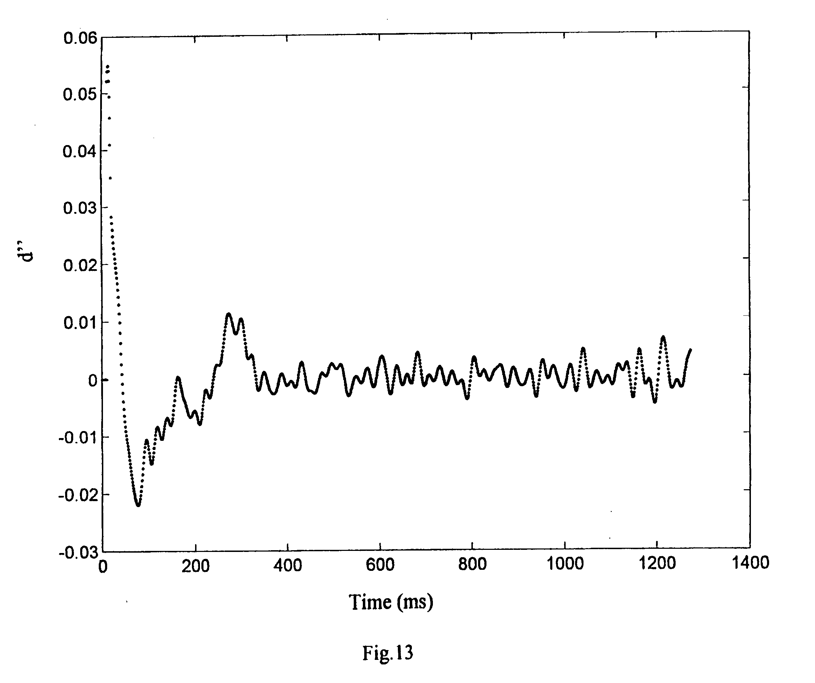

The above relation was applied in the cases where the pressure curve and the corresponding first and second derivatives, d' and d", were those shown in Figures 9 and 10.
ii) With -Zf3
In the cases where the pressure curves were of the type shown in Figure 11 and the corresponding first and second derivatives, d' and d", were as those shown in Figures 12 and 13, the relation became:

$$LSV = [k[A/((Zf1+Zf2-Zf3)^*1000)+A/((Zf1+Zf2-Zf3)^*1000)^*(Pm-K1)/K1]]/1000$$

Eq.[7]

where:

Zf3 = P3/(tfinal - t3); and

the symbols have the same meanings as specified previously, and t3 is the time, in [ms], of the minimum value of d" between the time tsys and the time tdic, and P3 is the corresponding pressure, expressed in [mmHg] at time t3 (see Figure 11).

$$LSVC = LSV+LSV^*abs ((Pd1-Pdic))/(psys-P(1))    Eq. [8]$$

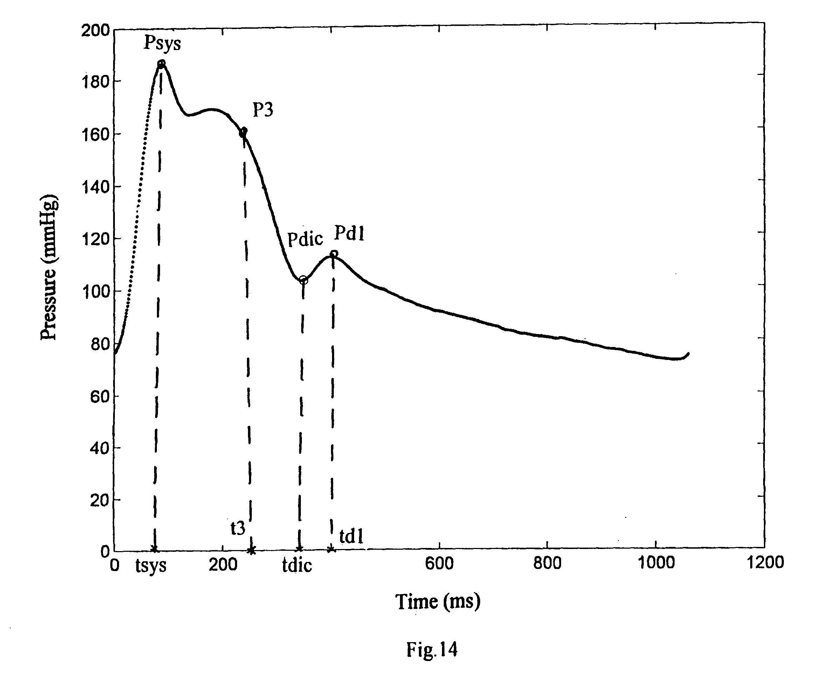

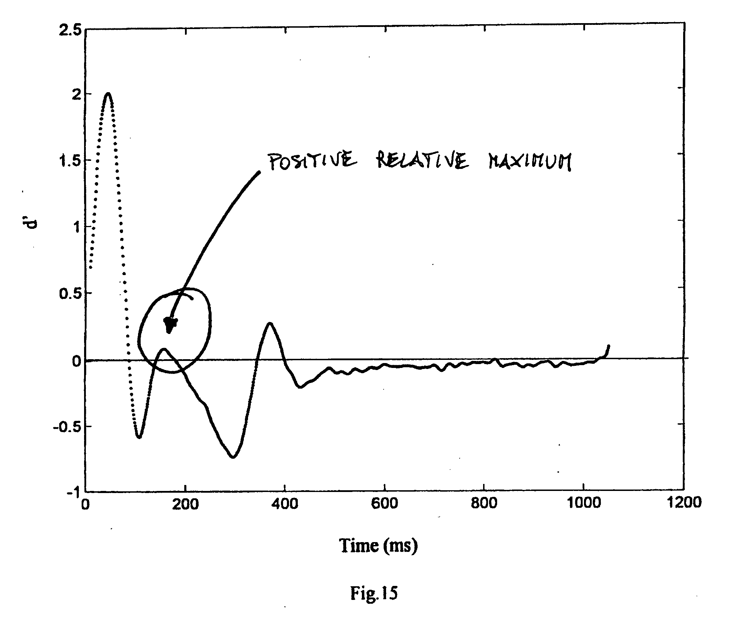

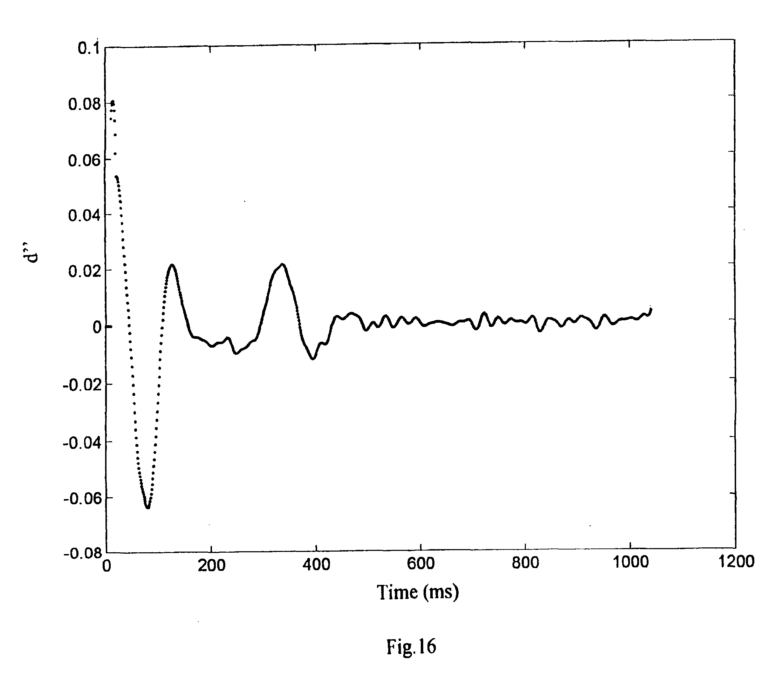

In a similar way it is possible to calculate Q = LSVC*HR.
iii) With -2Zf3
In the cases where the pressure curves were of the type shown in Figure 14 and the corresponding first and second derivatives, d' and d", were as those shown in Figures 15 and 16, the relation became:

$$LSV = [k[A/((Zf1+Zf2-2Zf3)^*1000)+A/((Zf1+Zf2-2Zf3)^*1000)^*(Pm-K1)/K1]]/1000$$

Eq.[9]

where Zf3 = P3/(tfinal - t3); and
the symbols have the same meanings as specified previously, and t3 is the time, expressed in [ms], of the minimum of d" between the time tsys and the time tdic, and P3 is the corresponding pressure, expressed in [mmHg] at time t3 (see Figure 14).

$$LSVC = LSV+LSV^*abs ((Pd1-Pdic))/(Psys-P1)    [10]$$

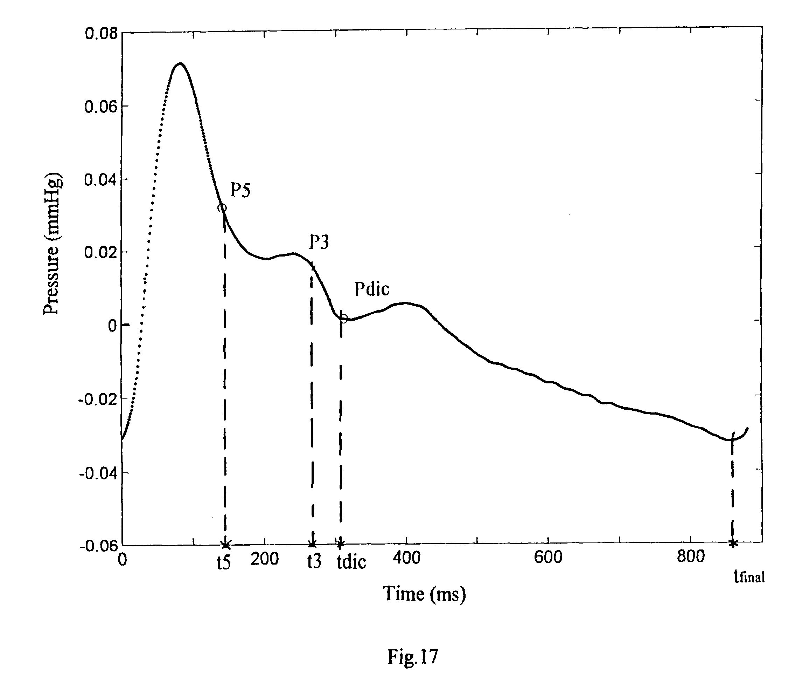

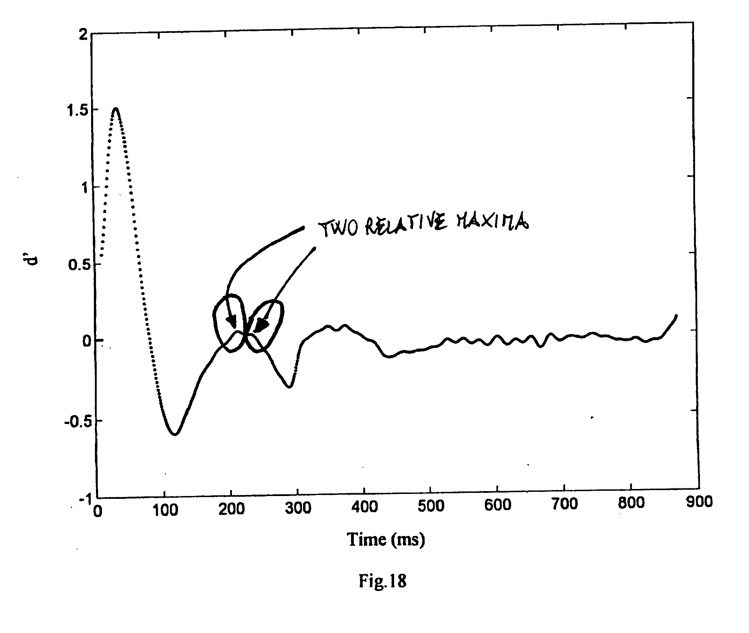

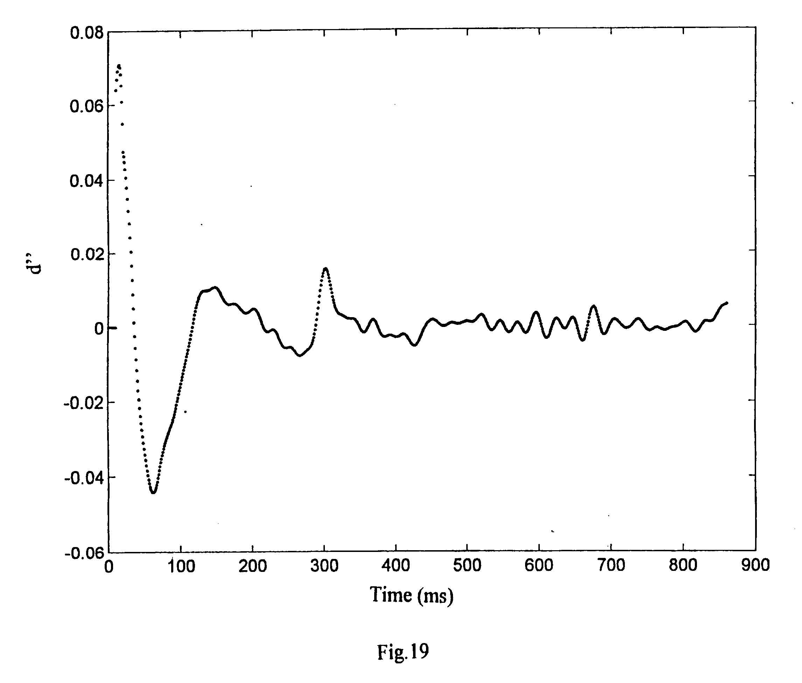

In a similar way it is possible to calculate Q = LSVC*HR, expressed in litres per minute.
iv) With -2Zf3-Zf5
In the cases where the pressure curves were of the type shown in Figure 17 and the corresponding first and second derivatives, d' and d", were as those shown in Figures 18 and 19, the relation became:

$$LSV = [k[A/((Zf1+Zf2-2Zf3-Zf5)*1000)+A/((Zf1+Zf2-2Zf3-Zf5)*1000)*(Pm-K1)/K1]]/1000$$

where Zf3 = P3/(tfinal - t3)
where Zf5 = P5/ (tfinale- t5)

the symbols have the same meanings as specified previously, and t5 is the time, expressed in [ms], of the minimum of d" between the time tsys and the time tdic, and P5 is the corresponding pressure, expressed in [mmHg] at time t5 (see Figure 17).

$$LSVC = LSV+LSV*abs\ ((P1-Pdic))/(Psys-P1)\qquad [11a]$$

In a similar way it is possible to calculate Q = LSVC*HR, expressed in litres per minute.
Note 3
The mean pressure in the case of the pressure taken at the arterial finger non invasively must be considered as such for the interval of mean pressure between 70 -110, for the values of mean pressure between 110 -150 and 70 - 40 mmHg it must be considered at 50% (for example a pm=128 for our method is = 119 mmHg); for mean values of pressure >150 and < 40 mmHg it must be considered at 25%
v) Reconstruction of the pressure signal in the ascending aorta by means of linear multiple regression in the time domain, by use of Zf1-Zf5

For these reconstruction, basically linear multiple regressions were used. In order to reconstruct the signal recorded in the ascending aorta (or in the pulmonary artery) using a cardiac catheter from the arterial signal recorded in a continuous way by means of a small cuff wrapped around the middle finger of the left hand, a linear multiple regression was used in which the reconstructed pressure signal was obtained in two successive steps:

1) An estimate was made of the mean pressure during the cardiac cycle in the ascending aorta (or in the pulmonary artery) from the signal taken at the finger, deriving the value Pmf (mean pressure in the aorta estimated from the recording taken at the finger) from the formulas used in the various cases of analysis of the arterial signal referred to in the previous points:

$$Pmf = LSV*Ztot/(k*A)\qquad [11b]$$

2) The waveform in the ascending aorta (or in the pulmomary artery) was reconstructed from a fit that used the following parameters:

$$y = a0*Pmf + a1*fin + a2*abs(derfin) + a3*abs(der2fin) + a4*abs(der3fin) +$$
$$a5*(intfin) + a6*slope*abs(derfin) + a7*slope*zZf1 + a8*slope$$
$$+ a9*maxfin + a10*minfin + a11*HR*(intfin(up\ to\ the\ point\ considered))+$$
$$a12*areaf + a13*zZf1 + a14*zZf2 + a15*zz3f + a16*zz4f\ a17*Zf5$$
$$areaf = cof*(Zf1+Zf2) / (Zf1+Zf2-n*z3f- Zf5)\qquad [12]$$

where

Zf5 and n = 0, 1 and 2 according to the criteria described previously;
zz4f = Pd1/ (tfinale- td1) (Figure 14) ;

- fin is the pressure at the finger;
- abs(derfin) is the absolute value of the first derivative in the pressure point considered;

- abs(der2fin) is the absolute value of the second derivative in the pressure point considered;
- abs(der3fin) is the absolute value of the third derivative in the pressure point considered;
- infin is the integral up to the point considered of the signal at the finger;
- slope is the angle between the horizontal axis and the straight line passing through the minimum points on the left and on the right of the cardiac cycle;
- maxfin and minfin coincide with the systolic pressure and the diastolic pressure;
- areaf is the total area of the pressure signal;

and the remaining symbols have the same meanings as specified previously.

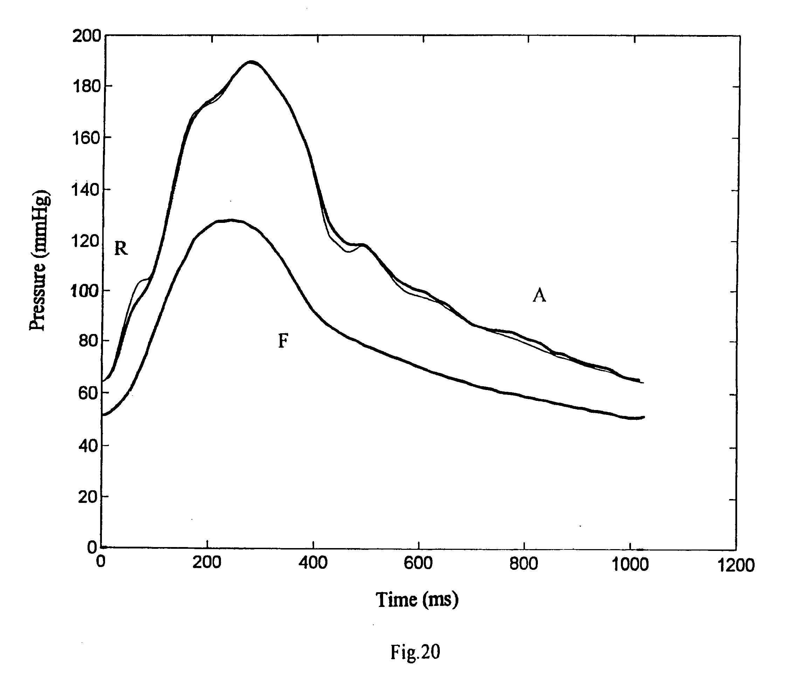

A number of reconstructions obtained are illustrated in Figure 20.

The errors in the comparison between the reconstructed curve of the signal registered non invasively and that taken directly near the ascending aortic are

| | SD(mmHg) | Max(mmHg) | Min(mmHg) |
|---|---|---|---|
| | 1:16÷5.67 | 2.38÷16.40 | -2.82÷-16.41 |
| | mean:3.41 | 9.37 | -9.32 |

With SD= Standard Deviation: the minimum of the interval is obtained for the riconstrruction of the points around the diastolic pressure, the maximum of the difference is obtained near the point of the systolic pressure.

Max= interval of over estimation of the pressure in the point taken into consideration reconstructed and that actually measured with the catheter during the cardiac beat the minimum of this interval is obtained for the reconstruction of the points around the diastolic pressure, the maximum of the difference is obtained near the points of systolic pressure.

Min= interval of under estimation of the pressure in the point taken into consideration reconstructed and that actually measured with the catheter during the cardiac beat the minimum of this interval is obtained for the reconstruction of the points around the diastolic pressure, the maximum of the difference is obtained near the points of systolic pressure.

What is important in this calculation are Zf1, Zf2, Zf3, Zf5, which we considered in point C) : these are necessary to have satisfactory results. D) Relation between LSV and the pressure recorded invasively from femoral artery or from another periferical point such as brachial or radial artery (Pulse Analytical Method, Brachial, Radial and Femoral, PAM (BRF)  ) ....

For these case we have seen that the formula to use are of the type used in the case C) non invasively with the following precisions: i) K1 for these invasive signals must be considered =100; ii) note 3 remains unchanged.

According to the invention, the method can be applied in combination with known methods (such as the thermodilution method) comprising a phase of calibration of the signal recorded, in which the contribution of the area under the pressure curve is considered variable over time, whereas the contribution of the impedance can only be considered constant.

In this case, the proposed method also makes it possible to take into account even major variations in the heart rate, in the pressure values and in the pressure waveform for purposes of calculation of the impedance.

It may therefore be concluded that both in the case of normal subjects and in the case of patients affected by various pathological conditions, the method proposed represents an effective and advantageous diagnostic tool in the invasive and non-invasive evaluation of cardiac output.

In addition, the method can be applied both in healthy subjects and in subjects presenting cardiocirculatory alterations who are undergoing ergometric tests that are aimed at establishing the level of haemodynamic response to the tests.

It should be emphasized that the present method is based only on the study of the pressure signal (recorded invasively in the pulmonary artery and in the aortic arch, or in any other major arterial vessel, or else non-invasively at the finger), and is independent of the anthropometric data and of the age of the subject examined.

**Claims**

1. Method for estimating cardiac output Q, comprising
   a first step of receiving a blood pressure, which has been acquired by means of a sensor, and converting the received blood pressure to a pressure signal p(t) expressed in mmHg;
   a second step of calculating an estimate of a left ventricular stroke volume LSV, such estimate being calculated as a function of an artery hydraulic impedance;
   a third step of calculating an estimate of the cardiac output Q as a product of the estimated left ventricular stroke volume LSV multiplied by a current heart rate value HR:

$$Q = LSV * HR.$$

the method being **characterised in that** the artery hydraulic impedance is calculated by means of an analysis of the first and second derivatives with respect to time of the pressure signal p(t) on the basis of the point of resonance, the systolic point, the diastolic point and the dicrotic point of the pressure signal p(t).

2. Method according to claim 1, **characterised in that** estimate of the left ventricular stroke volume LSV is calculated according to the following formula

$$LSV = (K/1000000) * [\, B\,(Pm)\,/K1] * (A/Ztot)]$$

where:

- K is a first constant equal to 1: K=1;
- Pm = (psys+2pdias)/3 is a mean pressure value, with
  psys = p(tsys) being the systolic pressure at the systolic pulse time tsys, and
  pdias = p(tdias) being the diastolic pressure at the diastolic pulse time tdias,
- wherein $B(P_m)$ is given, when said blood pressure is taken in an ascending aorta, by

$$B(Pm)=\begin{cases} 82,5 - 0,13 \cdot (70 - Pm) & \text{for} & Pm \le 70 \\[1.5em] 85 - 0,25 \cdot (80 - Pm) & \text{for} & 70 \le Pm \le 80 \\[1.5em] 90 - 0,5 \cdot (90 - Pm) & \text{for} & 80 \le Pm \le 90 \\[1.5em] Pm & \text{for} & 90 \le Pm \le 110 \\[1.5em] 110 + 0,5 \cdot (Pm - 110) & \text{for} & 110 \le Pm \le 120 \\[1.5em] 115 + 0,25 \cdot (Pm - 120) & \text{for} & 120 \le Pm \le 130 \\[1.5em] 117,5 + 0,13 \cdot (Pm - 130) & \text{for} & Pm \ge 130 \end{cases}$$

otherwise, when said blood pressure is taken in a pulmonary artery, by

$$B(Pm)=\begin{cases} 2 \cdot K1 & \text{for} & Pm < 19 \\[1.5em] Pm & \text{for} & 19 \le Pm \le 28 \\[1.5em] 28 + 0,5 \cdot (Pm - 28) & \text{for} & 28 \le Pm \le 33 \\[1.5em] 30,5 + 0,25 \cdot (Pm - 33) & \text{for} & Pm \ge 33 \end{cases}$$

otherwise, when said blood pressure is non-invasively recorded from the arterial finger or invasively recorded from femoral artery or from another periferal point such as brachial or radial artery, by

$$B(Pm)= \begin{cases} 55-0,25\cdot(40-Pm) & \textit{for} & Pm<40 \\ 70-0,5\cdot(70-Pm) & \textit{for} & 40\leq Pm<70 \\ Pm & \textit{for} & 70\leq Pm\leq 110 \\ 110+0,5\cdot(Pm-110) & \textit{for} & 110<Pm\leq 150 \\ 130+0,25\cdot(Pm-150) & \textit{for} & Pm>150 \end{cases}$$

- K1 is a second constant, equal to:

    100, when said blood pressure is taken in an ascending aorta or invasively recorded from a femoral artery or from another peripheral point such as brachial or radial artery, otherwise
    12, when said blood pressure is a taken in pulmonary artery, otherwise
    90, when said blood pressure is non-invasively recorded from the arterial finger,

- A is an area under the entire pressure signal p(t) given by the integral of the pressure signal p(t) between the time tdias at the diastolic pulse and the time tdic at the dicrotic pressure pdic, and
- Ztot is a dividing factor that is equal to
Z1 + Z2 - Z3,
when said blood pressure is taken in an ascending aorta and the pressure signal p(t) is of the type shown in figure 4, and the first and second derivatives of the pressure signal p(t) are as those shown in figures 5 and 6 as well as when said blood pressure is or non-invasively recorded from the arterial finger or invasively recorded from a femoral artery or from another peripheral point such as brachial or radial artery, and the pressure signal p(t) is of the type shown in figure 11, and the corresponding first and second derivatives of the pressure signal p(t) are as those shown in figure 12 and 13, or
Z1 + Z2 - 2*Z3,
when said blood pressure is non-invasively recorded from the arterial finger or invasively recorded from a femoral artery or another peripheral point such as brachial or radial artery and the pressure signal p(t) is of the type shown in figure 14, and the corresponding first and second derivatives of the pressure signal p(t) are as those shown in figures 15 and 16, or
Z1 +Z2 - 2*Z3 - Z5,
when said blood pressure is non-invasively recorded from the arterial finger or invasively recorded from a femoral artery or another peripheral point such as brachial or radial artery and the pressure signal p(t) is of the type shown in figure 17, and the corresponding first and second derivatives of the pressure signal p(t) are as those shown in figures 18 and 19,

where:

    - Z1 = (psys- pdias) / tsys,
    - Z2 = pdic/ (tfinal- tdic), with tfinal being the time of the end of the cardiac cycle,
    - Z3 = P3/ (tfinal- t3), with t3 being the time of the minimum value of the second derivative of the pressure signal p (t) between the time tsys and the time tdic, and P3=p (t3) being the corresponding pressure, and
    - Z5 = P5/ (tfinal- t5), with t5 being the time of the first maximum value of the second derivative of the pressure signal p (t) between the time tsys and the time tdic, and P5=p (t5) being the corresponding pressure.

3. Method according to claim 1, in which the pressure is a acquired in a non-invasive way from the arteriole of the finger, and a reconstruction is carried out of the pressure signal in the ascending aorta by means at a linear multiple regression.

4. Apparatus for estimating cardiac output Q, **characterised in that** it comprises:

- pressure sensing means for sensing a blood pressure; and
- processing means for carrying out methods according to any one of claims 1-3.

5. Apparatus according to claim 4, **characterised in that** the pressure sensing means is a small cuff for non-invasive measurements, which is wrapped around a finger.

6. Computer program loadable in a computer unit, **characterised in that** it is adapted to execute, when running on the computer unit, the method for estimating cardiac output Q according to any one of the preceding claims 1-3.

7. A computer-readable medium storing a computer program, **characterised in that** the program is the computer program according to claim 6.

**Patentansprüche**

1. Verfahren zum Schätzen einer Herzleistung Q, wobei das Verfahren Folgendes umfasst:

einen ersten Schritt des Empfangens eines Blutdrucks, der mittels eines Sensors erfasst wurde, und des Umwandelns des empfangenen Blutdrucks in ein Drucksignal p(t), das in mmHg ausgedrückt ist,
einen zweiten Schritt des Berechnens einer Schätzung eines linksventrikulären Hubvolumens LSV, bei dem die Schätzung als eine Funktion eines arteriellen Strömungswiderstandes berechnet wird,
einen dritten Schritt des Berechnens einer Schätzung der Herzleistung Q als ein Produkt des geschätzten linksventrikulären Hubvolumens LSV multipliziert mit einem aktuellen Herzfrequenzwert HR:

$$Q = LSV * HR,$$

wobei das Verfahren **dadurch gekennzeichnet ist, dass** der arterielle Strömungswiderstand mittels einer Analyse der ersten und zweiten zeitlichen Ableitung des Drucksignals p(t) auf der Basis des Resonanzpunktes, des systolischen Punktes, des diastolischen Punktes und des dikroten Punktes des Drucksignals p(t) berechnet wird.

2. Verfahren gemäß Anspruch 1, **dadurch gekennzeichnet, dass** die Schätzung des linksventrikulären Hubvolumens LSV gemäß der folgenden Formel berechnet wird:

$$LSV = (K/1000000) * [B(Pm)/K1] * (A/Ztot)],$$

wobei:

K eine erste Konstante gleich 1 ist: K = 1,
Pm = (psys + 2 pdias) / 3 ein mittlerer Druckwert ist, wobei psys = p(tsys) der systolische Druck bei der systolischen Pulszeit tsys ist, und
pdias = p(tdias) der diastolische Druck bei der diastolischen Pulszeit tdias ist,
wobei B(Pm), wenn der Blutdruck in einer aufsteigenden Aorta gemessen wird, gegeben ist durch

$$
B(Pm) = \begin{cases}
82{,}5 - 0{,}13 \bullet (70 - Pm) & \text{für} & Pm \leq 70 \\
85 - 0{,}25 \bullet (80 - Pm) & \text{für} & 70 \leq Pm \leq 80 \\
90 - 0{,}5 \bullet (90 - Pm) & \text{für} & 80 \leq Pm \leq 90 \\
Pm & \text{für} & 90 \leq Pm \leq 100 \\
110 + 0{,}5 \bullet (Pm - 110) & \text{für} & 110 \leq Pm \leq 120 \\
115 + 0{,}25 \bullet (Pm - 120) & \text{für} & 120 \leq Pm \leq 130 \\
117{,}5 + 0{,}13 \bullet (Pm - 130) & \text{für} & Pm \geq 130
\end{cases}
$$

und andernfalls, wenn der Blutdruck in einer Pulmonalarterie gemessen wird, durch

$$
B(Pm) = \begin{cases}
2 \bullet K1 & \text{für} & Pm < 19 \\
Pm & \text{für} & 19 \leq Pm \leq 28 \\
28 + 0{,}5 \bullet (Pm - 28) & \text{für} & 28 \leq Pm \leq 33 \\
30{,}5 + 0{,}25 \bullet (Pm - 33) & \text{für} & Pm \geq 33
\end{cases}
$$

andernfalls, wenn der Blutdruck nicht-invasiv von dem arteriellen Finger aufgezeichnet wird oder invasiv von einer Femoralarterie oder von einem anderen peripheren Punkt, wie beispielsweise einer Brachial- oder Radialarterie aufgezeichnet wird, durch

$$
B(Pm) = \begin{cases}
55 - 0{,}25 \bullet (40 - Pm) & \text{für} & Pm < 40 \\
70 - 0{,}5 \bullet (70 - Pm) & \text{für} & 40 \leq Pm < 70 \\
Pm & \text{für} & 70 \leq Pm \leq 110 \\
110 + 0{,}5 \bullet (Pm - 110) & \text{für} & 110 < Pm \leq 150 \\
130 + 0{,}25 \bullet (Pm - 150) & \text{für} & Pm > 150
\end{cases}
$$

wobei K1 eine zweite Konstante ist, die gleich

100 ist, wenn der Blutdruck in einer aufsteigenden Aorta gemessen wird oder invasiv von einer Femoralarterie oder von einem anderen peripheren Punkt, wie beispielsweise einer Brachial- oder Radialarterie aufgezeichnet wird, andernfalls
12 ist, wenn der Blutdruck in einer Pulmonalarterie gemessen wird, andernfalls 90 ist, wenn der Blutdruck nicht-invasiv von dem arteriellen Finger aufgezeichnet wird,

wobei A eine Fläche unter dem gesamten Drucksignal p(t) ist, gegeben durch das Integral des Drucksignals p (t) zwischen der Zeit tdias bei dem diastolischen Puls und der Zeit tdic bei dem dikroten Druck pdic, und wobei Ztot ein Teilungsfaktor ist, der gleich
Z1 + Z2 - Z3 ist, wenn der Blutdruck in einer aufsteigenden Aorta gemessen wird und das Drucksignal p(t) von dem Typ ist, der in Figur 4 gezeigt ist, und die erste und die zweite Ableitung des Drucksignals p(t) wie diejenigen sind, die in den Figuren 5 und 6 gezeigt sind, sowie wenn der Blutdruck nicht-invasiv von dem arteriellen Finger aufgezeichnet wird oder invasiv von einer Femoralarterie oder von einem anderen peripheren Punkt, wie beispielsweise einer Brachial- oder Radialarterie aufgezeichnet wird, und das Drucksignal p(t) von dem Typ ist, der in Figur 11 gezeigt ist, und die entsprechende erste und zweite Ableitung des Drucksignals p(t) wie diejenigen sind, die in Figur 12 und 13 gezeigt sind, oder
Z1 + Z2 - 2*Z3 ist, wenn der Blutdruck nicht-invasiv von dem arteriellen Finger aufgezeichnet wird oder invasiv von einer Femoralarterie oder einem anderen peripheren Punkt, wie beispielsweise einer Brachial- oder Radialarterie aufgezeichnet wird, und das Drucksignal p(t) von dem Typ ist, der in Figur 14 gezeigt ist, und die entsprechende erste und zweite Ableitung des Drucksignals p(t) wie diejenigen sind, die in den Figuren 15 und 16 gezeigt sind, oder

Z1 + Z2 - 2*Z3 - Z5 ist, wenn der Blutdruck nicht-invasiv aus dem arteriellen Finger aufgezeichnet wird oder invasiv aus einer Femoralarterie oder einem anderen peripheren Punkt, wie beispielsweise einer Brachial- oder Radialarterie aufgezeichnet wird, und das Drucksignal p(t) von dem Typ ist, der in Figur 17 gezeigt ist, und die entsprechende erste und zweite Ableitung des Drucksignals p(t) wie diejenigen sind, die in den Figuren 18 und 19 gezeigt sind,

wobei

Z1 = (psys - pdias) / tsys ist,

Z2 = pdic / (tfinal - tdic) ist, wobei tfinal die Zeit des Endes des Herzzyklus ist,

Z3 = P3 / (tfinal - t3) ist, wobei t3 die Zeit des minimalen Wertes der zweiten Ableitung des Drucksignals p(t) zwischen der Zeit tsys und der Zeit tdic ist, und wobei P3 = p(t3) der entsprechende Druck ist, und

Z5 = P5 / (tfinal - t5) ist, wobei t5 die Zeit des ersten maximalen Wertes der zweiten Ableitung des Drucksignals p(t) zwischen der Zeit tsys und der Zeit tdic ist, und wobei P5 = p(t5) der entsprechende Druck ist.

3. Verfahren gemäß Anspruch 1, wobei der Druck auf nicht-invasive Art von der Arteriole des Fingers erfasst wird, und eine Rekonstruktion des Drucksignals in der aufsteigenden Aorta mittels einer linearen multiplen Regression durchgeführt wird.

4. Vorrichtung zum Schätzen einer Herzleistung Q, **dadurch gekennzeichnet, dass** sie Folgendes umfasst:

   ein Druckerfassungsmittel zum Erfassen eines Blutdrucks und
   ein Verarbeitungsmittel zum Durchführen von Verfahren gemäß einem der Ansprüche 1 bis 3.

5. Vorrichtung gemäß Anspruch 4, **dadurch gekennzeichnet, dass** das Druckerfassungsmittel eine kleine Manschette für nicht-invasive Messungen ist, welche um einen Finger gewickelt ist.

6. Computerprogramm, das in eine Computereinheit ladbar ist, **dadurch gekennzeichnet, dass** es dazu eingerichtet ist, das Verfahren zum Schätzen einer Herzleistung Q gemäß einem der vorhergehenden Ansprüche 1 bis 3 durchzuführen, wenn es auf der Computereinheit abläuft.

7. Computerlesbares Medium, auf welchem ein Computerprogramm gespeichert ist, **dadurch gekennzeichnet, dass** das Programm das Computerprogramm gemäß Anspruch 6 ist.

**Revendications**

1. Procédé pour estimer un débit cardiaque Q, comprenant
une première étape de réception d'une pression artérielle, qui a été acquise au moyen d'un détecteur, et de conversion de la pression artérielle reçue en un signal de pression p(t) exprimé en mmHg ;
une deuxième étape de calcul d'une estimation d'un volume d'éjection ventriculaire gauche LSV, une telle estimation étant calculée en fonction de l'impédance hydraulique artérielle;
une troisième étape de calcul d'une estimation du débit cardiaque Q en tant que produit du volume d'éjection ventriculaire gauche LSV estimé multiplié par une valeur de fréquence cardiaque réelle HR :

$$Q = LSV * HR.$$

le procédé étant **caractérisé en ce que** l'impédance hydraulique artérielle est calculée au moyen d'une analyse des première et deuxième dérivées par rapport au temps du signal de pression p(t) en se basant sur le point de résonance, le point systolique, le point diastolique et le point dicrote du signal de pression p(t).

2. Procédé selon la revendication 1, **caractérisé en ce que** l'estimation du volume d'éjection ventriculaire gauche LSV est calculée selon la formule suivante

$$LSV = (K/1000000)*[B(Pm)/K1]*(A/Ztot)]$$

où :

- K est une première constante égale à 1 : K=1 ;
- Pm = (psys+2pdias)/3 est une valeur de pression moyenne, avec psys = p(tsys) étant la pression systolique au temps d'impulsion systolique tsys, et pdias = p(tdias) étant la pression diastolique au temps d'impulsion diastolique tdias,
- où B(Pm) est donnée, où ladite pression artérielle est prisse dans une aorte ascendante
par

$$B(Pm)=$$
$$\begin{cases} 82,5 - 0,13 \cdot (70 - Pm) & pour & Pm \leq 70 \\ 85 - 0,25 \cdot (80 - Pm) & pour & 70 \leq Pm \leq 80 \\ 90 - 0,5 \cdot (90 - Pm) & pour & 80 \leq Pm \leq 90 \\ Pm & pour & 90 \leq Pm \leq 110 \\ 110 + 0,5 \cdot (Pm - 110) & pour & 110 \leq Pm \leq 120 \\ 115 + 0,25 \cdot (Pm - 120) & pour & 120 \leq Pm \leq 130 \\ 117,5 + 0,13 \cdot (Pm - 130) & pour & Pm \geq 130 \end{cases}$$

autrement, lorsque ladite pression artérielle est prise dans une artère pulmonaire par

$$B(Pm)=$$
$$\begin{cases} 2 \cdot K1 & pour & Pm < 19 \\ Pm & pour & 19 \leq Pm \leq 28 \\ 28 + 0,5 \cdot (Pm - 28) & pour & 28 \leq Pm \leq 33 \\ 30,5 + 0,25 \cdot (Pm - 33) & pour & Pm \geq 33 \end{cases}$$

autrement, lorsque ladite pression artérielle est enregistrée de manière non invasive à partir de l'artère du doigt, ou est enregistrée de manière invasive à partir de l'artère fémorale ou d'un autre point périphérique comme l'artère brachiale ou radiale, par

$$B(Pm)=$$
$$\begin{cases} 55 - 0,25 \cdot (40 - Pm) & pour & Pm < 40 \\ 70 - 0,5 \cdot (70 - Pm) & pour & 40 \leq Pm < 70 \\ Pm & pour & 70 \leq Pm \leq 110 \\ 110 + 0,5 \cdot (Pm - 110) & pour & 110 < Pm \leq 150 \\ 130 + 0,25 \cdot (Pm - 150) & pour & Pm > 150 \end{cases}$$

- K1 est une deuxième constante, égale à :

100, lorsque ladite pression artérielle est prise dans une aorte ascendante ou est enregistrée de manière invasive à partir d'une artère fémorale ou à partir d'un autre point périphérique comme l'artère brachiale ou radiale, autrement

12, lorsque ladite pression artérielle est prisse dans une artère pulmonaire, autrement 90, lorsque ladite pression artérielle est enregistrée de manière non invasive à partir de l'artère du doigt

- A est une surface sous l'intégralité du signal de pression p(t) donnée par l'intégrale du signal de pression p(t) entre le temps tdias à l'impulsion diastolique et le temps tdic à la pression dicrote pdic, et
- Ztot est un facteur de division qui est égal à
Z1 + Z2 - Z3,
lorsque ladite pression artérielle est prise dans une aorte ascendante et que le signal de pression p(t) est du type montré dans la figure 4, et les première et deuxième dérivées du signal de pression p(t) sont telles que celles montrées dans les figures 5 et 6, ainsi que lorsque ladite pression artérielle est enregistrée de manière non invasive à partir de l'artère du doigt ou est enregistrée de manière invasive à partir d'une artère fémorale ou à partir d'un autre point périphérique comme l'artère brachiale ou radiale, et que le signal de pression p(t) est du type montré dans la figure 11, et les première et deuxième dérivées correspondantes du signal de pression p(t) sont telles que celles montrées dans les figures 12 et 13, ou

$Z1 + Z2 - 2*Z3$,

lorsque ladite pression artérielle est enregistrée de manière non invasive à partir de l'artère du doigt ou est enregistrée de manière invasive à partir d'une artère fémorale ou d'un autre point périphérique comme l'artère brachiale ou radiale, et que le signal de pression p(t) est du type montré dans la figure 14, et les première et deuxième dérivées correspondantes du signal de pression p(t) sont telles que celles montrées dans les figures 15 et 16, ou

$Z1 + Z2 - 2*Z3 - Z5$,

lorsque ladite pression artérielle est enregistrée de manière non invasive à partir de l'artère du doigt ou est enregistrée de manière invasive à partir d'une artère fémorale ou d'un autre point périphérique comme l'artère brachiale ou radiale, et que le signal de pression p(t) est du type montré dans la figure 17, et les première et deuxième dérivées correspondantes du signal de pression p(t) sont telles que celles montrées dans les figures 18 et 19,

où :
- $Z1 = (p_{sys} - p_{dias}) / t_{sys}$,
- $Z2 = p_{dic} / (t_{final} - t_{dic})$, $t_{final}$ étant le temps de la fin du cycle cardiaque,
- $Z3 = P3 / (t_{final} - t3)$, $t3$ étant le temps de la valeur minimale de la deuxième dérivée du signal de pression p(t) entre le temps $t_{sys}$ et le temps $t_{dic}$, et $P3 = p(t3)$ étant la pression correspondante, et
- $Z5 = P5 / (t_{final} - t5)$, $t5$ étant le temps de la première valeur maximale de la deuxième dérivée du signal de pression p(t) entre le temps $t_{sys}$ et le temps $t_{dic}$, et $P5 = p(t5)$ étant la pression correspondante.

3. Procédé selon la revendication 1, dans lequel la pression est acquise de manière non invasive à partir de l'artériole du doigt, et une reconstruction du signal de pression dans l'aorte ascendante est réalisée au moyen d'une régression linéaire multiple.

4. Appareil pour estimer le débit cardiaque Q, **caractérisé en ce qu'**il comprend :

   - un moyen de détection de la pression pour détecter une pression artérielle ; et
   - un moyen de traitement pour réaliser les procédés selon l'une quelconque des revendications 1 à 3.

5. Appareil selon la revendication 4, **caractérisé en ce que** le moyen de détection de la pression est une petite manchette pour des mesures non invasives, qui est enroulée autour d'un doigt.

6. Programme informatique pouvant être chargé sur une unité informatique, **caractérisé en ce qu'**il est adapté pour exécuter, lorsqu'il est lancé sur l'unité informatique, le procédé d'estimation du débit cardiaque Q selon l'une quelconque des revendications précédentes 1 à 3.

7. Support lisible par un ordinateur stockant un programme informatique, **caractérisé en ce que** le programme est le programme informatique selon la revendication 6.

Fig. A1

EP 1 173 093 B1

Fig.1

Fig.2

Fig.3

EP 1 173 093 B1

Fig.4

EP 1 173 093 B1

Fig.5

Fig.6

Fig.7

Fig.8

EP 1 173 093 B1

Fig.9

Fig.10

Time (ms)

d''

Fig.11

Fig.12

EP 1 173 093 B1

EP 1 173 093 B1

Fig.13

Fig.14

EP 1 173 093 B1

Fig.15

Fig.16

EP 1 173 093 B1

Fig.17

Fig.18

Fig.19

EP 1 173 093 B1

Fig.20

Time (ms)

Pressure (mmHg)

A

F

R

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5647369 A